# EUROPEAN PATENT SPECIFICATION

(11) **EP 0 349 545 B1**
(45) Date of publication and mention of the grant of the patent: **03.02.1993**
(21) Application number: 88901838.8
(22) Date of filing: 27.02.1988
(51) Int. Cl.: C07K 7/10, A61K 37/24, C07K 1/04, G01N 33/53, A61K 35/34

(54) **NEW CARDIODILATIN FRAGMENT, PROCESS FOR PREPARING SAME AND USE THEREOF**
NEUES CADIODILATIN-FRAGMENT, PROZESS ZU DESSEN HERSTELLUNG UND DESSEN ANWENDUNG
NOUVEAU FRAGMENT DE CARDIODILATINE, SON PROCEDE DE PREPARATION ET SON EMPLOI

(30) Priority: 02.03.1987 DE 3706731; 22.05.1987 DE 3717329; 09.12.1987 DE 3741641
(43) Date of publication of application: 10.01.1990
(73) Proprietor: FORSSMANN, Wolf-Georg, Prof. Dr. med., D-69121 Heidelberg (DE); PHARMA BISSENDORF PEPTIDE GMBH, 30625 Hannover (DE)
(72) Inventor: FORSSMANN, Wolf-Georg, D-6900 Heidelberg 1 (DE); ALT, Jeanette, M., D-3006 Burgwedel 3 (DE); BECKER, Gerhard, D-6903 Neckargemünd 3 (DE); HERBST, Franz, D-6906 Leimen (DE)
(74) Representative: Werner, Hans-Karsten, Dr.Dipl.-Chem.
(86) International application number: EP8800144
(87) International publication number: WO8806596

(56) References cited:
- EP-A- 0 182 984
- WO-A-85/04870
- Biochemical and Biophysical Research Communications, vol. 124, no. 3, 14 november 1984, Academic Press. Inc. K. Nakao et al.: "Radioimmunoassay for -human and rat atrial natriuretic polypeptide", pages 815-821, see the whole disclosure
- Biochemical and Biophysical Research Communications, vol. 121, no. 2, 15 June 1984, Academic Press. Inc. K. Kangawa et al.: "Identification in rat atrial tissue of multiple forms of natriuretic polypeptides of about 3,000 daltons", bladzijde 585-591, see page 590, figure 3; exposure compound D
- Proceedings of the National Academy of Sciences, vol. 81, no. 9, May 1984, Biochemistry N.G. Seidah et al.: "Amino acid sequence of homologous rat atrial peptides: Natriuretic activity of native and synthetic forms", pages 2640-2644, see page 2641, figures 1,2; page 2642, figure 3
- Science, vol. 226, 10 September 1984 C.E. Seidman: "Nucleotide sequences of the human and mouse atrial natriuretic factor genes", pages 1206 - 1209, see page 1208, figure 3

## Description

The invention relates to a new cardiodilatin fragment, a process for preparing same and a medicament containing said fragment.

Biologically active peptides such as, more particularly, hormones are produced in low amounts in various endocrine glands and are transported via the blood to the target organs. There they control quite a number of physiological and metabolic activities. As a rule, inactive forms of the respective peptides are sythesized in the respective endocrine gland and are converted into their active forms under the action of proteases. Only in such active forms they exert the desired effect on the target organ.

In the last years atrial extracts from rat atria have been thoroughly investigated. As a result, a number of different peptides has been isolated which display stronger or lesser sodiuretic and diuretic effects and which additionally are capable of relaxing the smooth muscles of vessels. The nomenclature of these peptides so far was rather confusing (atrial sodiuretic factor, auriculin A, cardionatrin, auriculin B, atriopeptin I, II and III, gamma- and alpha-atrial sodiuretic factor). It is to be assumed that this variety of terms in part was caused by preparative artefacts, and that, on the other hand, different peptides were prepared from a common precursor. A common precursor of the human peptides as well as of the peptides of rats could be prepared from cloned cDNS, which had been prepared from atrial mRNA. The human prepropeptide has 151 amino acids. The first 25 amino acids of this peptide correspond to the prepropeptide portion which is designated as signal peptide and controls processing along the ribosomal synthesis and the subsequent secretion. Thereafter a peptid comprising 126 amino acids, the ANF/CDD 1-126 (gamma-hANaP), is released which has a molecular weight of 13,500 and in this form has also been identified in human atria.

An essential biologically active peptide corresponds to the last 28 amino acids at the C-terminal end of ANF/CDD 1-126 (gamma-hANaP). This peptide has been designated as ANF/CDD 99-126 (alpha-human atrial sodiuretic peptide, alpha-hANaP). The amino acid sequence has been elucidated and published by Kangawa, K. and Matsuo, cf. Biochem. Biophys. Res. Commun. 118, 131-139 (1984). 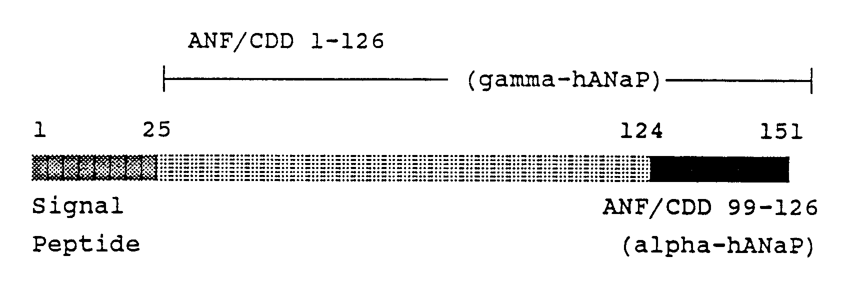
ANF/CDD 1-126
(gamma-hANaP)
1 25 124 151
Signal ANF/CDD 99-126
Peptide (alpha-hANaP)
The positions 1-25 of the amino acid sequence are considered as the signal peptide, whereas the positions 25-151 represent the gamma-hANaP, which inter alia in the region of the C-terminal end (positions 124-151) is identical with the alpha-hANaP (Oikawa, S. et al., "Nature" 309 (1984), 724-726; Nakayama, K. et al., "Nature" 310 (1984), 699-701). However, the numbering as used hereinbelow will leave the signal peptide out of consideration. Thus, there is valid for gamma-hANaP an amino acid sequence of positions 1-126, and for Alpha-hANaP an amino acid sequence of positions 99-126 (ANF/CDD 99-126).

This peptide hormone which is also known under the designation of cardiodilatin is of great clinical and therapeutical importance because of its influence on renal diuresis of the inotropy of the cardiac muscle and on the smooth musculature of the vessels as well as the perspiration. Some fragments of this peptide hormone exhibit biological activity, in part in an attenuated form. ANF/CDD 99-126 is an active fragment of cardiodilatins which circulates in the blood. It has the following amino acid sequence: 
Ser-Leu-Arg-Arg-Ser-Ser-Cys-Phe-Gly-Gly-Arg-Met-Asp-
Arg-Ile-Gly-Ala-Gln-Ser-Gly-Leu-Gly-Cys-Asn-Ser-Phe-
Arg-Tyr-OH.
The ANF/CDD 99-126 may be obtained from atrium extracts only in infinitely small amounts.

It is the object of the present invention to find further biologically active fragments of cardiodilatin and further to create processes for the synthetic preparation of this biologically active cardiodilatin fragment.

Said object is attained by the cardiodilatin fragment urodilatin (ANF/CDD 95-126) which has the amino acid sequence 
R1-Cys-Phe-Gly-Gly-Arg-Met-
S
S
Asp-Arg-Ile-Gly-Ala-Gln-Ser-Gly-Leu-Gly-Cys-R2,
wherein R¹ and R² each represent further peptide fragments of ANF/CDD 1-126 (gamma-hANaP) and, more particularly
R¹ is Thr-Ala-Pro-Arg-Ser-Leu-Arg-Arg-Ser-Ser and
R² is Asn-Ser-Phe-Arg-Tyr.

Surprisingly it was found that human urine contains a so far unknown biologically active form of cardiodilatin - urodilatin (ANF/CDD 95-126). Animal tests with rats and dogs showed that urodilatin (ANF/CDD 95-126), although it displays an action corresponding to that of ANF/CDD 99-126 only at a somewhat higher dose as compared to ANF/CDD 99-126, has a clearly stronger diuretic effect. This biologically active material is eluting in certain steps of its isolation in a manner significantly different from ANF/CDD 99-126 in the chromatographic system used for the isolation of cardiodilatin fragments.

It was found that urodilatin (ANF/CDD 95-126) originates from precursor proteins formed in the kidneys by post-translatoric processing. 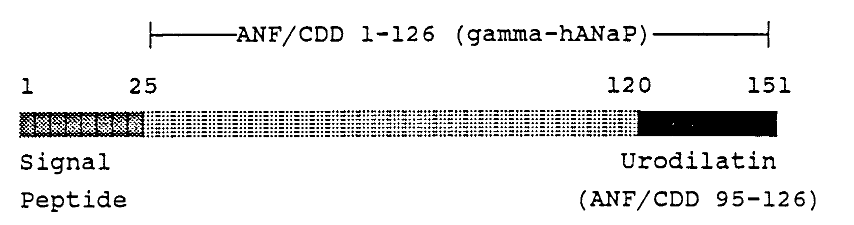
ANF/CDD 1-126 (gamma-hANaP)
1 25 120 151
Signal Urodilatin
Peptide (ANF/CDD 95-126)
The positions 1-25 of the amino acid sequence of the prepropeptide are considered as the signal peptide, whereas the positions 25-151 represent the ANF/CDD 1-126 (gamma-hANaP) (Oikawa, S. et al., "Nature" 309 (1984), 724-726; Nakayama, K. et al., "Nature" 310 (1984), 699-701), which in the region of the C-terminal end (positions 120-151) is identical with the urodilatin (ANF/CDD 95-126). However, the numbering as used hereinbelow will leave the signal peptide out of consideration. Thus, there is valid for ANF/CDD 1-126 (gamma-hANAP) an amino acid sequence of positions 1-126, and for urodilatin (ANF/CDD 95-126) an amino acid sequence of positions 95-126.

This new cardiodilatin fragment which has a surprising biological activity and is distinguished from ANF/CDD 99-126, was isolated from human urine in an amount sufficient for elucidating its structure and was obtained in a particularly pure state by means of HPLC techniques, so that as a singulary molecule its structure could be elucidated by an amino acid sequence analysis. The amino acid sequence of this fragment was:

Thr-Ala-Pro-Arg-Ser-Leu-Arg-Arg-Ser-Ser-Cys-Phe-Gly-
Gly-Arg-Met-Asp-Arg-Ile-Gly-Ala-Gln-Ser-Gly-Leu-Gly-
Cys-Asn-Ser-Phe-Arg-Tyr.

In vivo- and in vitro investigations on the activities of urodilatin (ANF/CDD 95-126) show that this substance has a marked relaxant effect on the smooth musculature. In vitro investigations of the Arteria renalis of rabbits, of the Aorta abdominalis of rabbits or rats or of the Arteria pulmonalis of rabbits in the organ bath, which have been contracted by adrenalin pretreatment, also show a relaxant effect of the urodilatin (ANF/CDD 95-126). Most clearly evident is this relaxation in muscle stripes from Arteria renalis. There the relaxation is already detectable at doses of about 1.0 to 10.00 mg per 10 ml in the organ bath. Urodilatin (ANF/CDD 95-126) acts vasodilative as well as diuretic and sodiuretic.

While ANF/CDD 99-126 may be isolated from material obtained in hemodialysis or hemofiltration, respectively, urodilatin may be isolated from human urine. For a recovery of urodilatin (ANF/CDD 95-126) urine, if appropriate after acidification, is first contacted with alginic acid. The cardiodilatin fragments will be adsorbed on the alginic acid. The work-up and isolation of the cardiodilatin fragments by detaching same from the alginic acid and chromatographic purification are carried out as already described in the DE-OS 33 46 953 for the total molecule ANF/CDD 1-126 (gamma-hANaP).

For effecting still further purification of the desired biologically active cardiolatin fragment, the fraction containing the biologically active molecule may be subjected to a second separation by HPLC using a reversed-phase chromatography silicagel. In this second chromatography step it is preferred to use a mixture comprising water, methanol and trifluoroacetic acid with a continuous gradient as eluant.

This new cardiodilatin fragment urodilatin (ANF/CDD 95-126) may also be synthesized in a per se known manner, e.g. by Merrifield synthesis using BOC amino acids. A preparation is also possible by way of a classical fragment synthesis in solution.

Another subject matter of the invention is a process for the preparation of the cardiodilatin fragment urodilatin (ANF/CDD 95-126) having the amino acid sequence: 
R1-Cys-Phe-Gly-Gly-Arg-Met-
S
S
Asp-Arg-Ile-Gly-Ala-Gln-Ser-Gly-Leu-Gly-Cys-R2,
wherein R¹ and R² each represent further peptide fragments of ANF/CDD 1-126 (gamma-hANaP) and, more particularly
R¹ is Thr-Ala-Pro-Arg-Ser-Leu-Arg-Arg-Ser-Ser and
R² is Asn-Ser-Phe-Arg-Tyr,
by stepwise synthesis on a solid phase using amino acid protected with conventional protective groups.

As the protective groups there are preferably used tert-butyloxycarbonyl, fluorenylmethoxycarbonyl or 3,5-dimethoxyphenyl-2,2-propyloxycarbonyl groups.

In a further embodiment of the process according to the invention, ANF/CDD 99-126 is directly reacted with the N-terminally extending tetrapeptide Thr-Ala-Pro-Arg-OH. Since the peptide ANF/CDD 99-126 does not contain pendant groups having free amino functions, this synthesis may be carried out relatively free from problems. The tetrapeptide Thr-Ala-Pro-Arg is previously synthesized in a per se known manner and reacted, if appropriate in its protected form, with free ANF/CDD 99-126 after activation of its carboxyl functions.

Another embodiment of the process according to the invention for the preparation of the cardiodilatin fragment urodilatin (ANF/CDD 95-126) is characterized in that ANF/CDD 99-126, which has been immobilized on a solid phase, is reacted with the tetrapeptide Thr-Ala-Pro-Arg which, if appropriate, contains protective group(s), the resulting peptide is detached from the carrier, subjected to cyclization upon removal of the protective group(s) and worked up and purified in a per se known manner.

The tetrapeptide may have been protected by conventional protective groups. Preferably used tetrapeptides are Fmoc-Thr(But)-Ala-Pro-Arg(Mtr) or Ddz-Thr(But)-Ala-Pro-Arg(Mtr). The pendant group of Arg in the tetrapeptide may also be present protected in the salt form as bromide or perchlorate.

As an activating agent there is preferred to be used carbonyldiimidazol with 2 equivalents of 1-hydroxybenzotriazole.

A further subject matter of the invention is a process for the preparation of the cardiodilatin fragment urodilatin (ANF/CDD 95-126) which process is characterized in that the peptide ANF/CDD 99-126 is dissolved in a solvent, the tetrapeptide Thr-Ala-Pro-Arg, provided with protective group(s) if appropriate, is added together with an activating agent, and the resulting product is subjected to chromatography after removal of the protective groups and further purified in a per se known manner.

The urodilatin (ANF/CDD 95-126) having been completely synthetically prepared by means of the solid phase synthesis method according to Merrifield (R.B., "Solid Phase Peptide Synthesis I, The Synthesis of a Tetrapeptide", J. Am. Chem. Soc. 85, 2149-2156, (1963)) using BOC amino acids is obtained as a very pure substance (purity more than 98%) and in a very active form.

The synthesis of urodilatin (ANF/CDD 95-126) may be carried out by analogy of the solid phase synthesis method of Merrifield (1963) by means of benzhydrylamine as a supporting polymer in an automatic peptide synthesizer. The crude preparation is prepurified over Sephadex-G 25 (F) and subsequently purified by means of a semi-preparative high pressure liquid chromatography (HPLC). Thus, the fully synthetic replicate of the last C-terminal amino acids (95 to 126) of ANF/CDD 1-126 (gamma-hANaP) is formed. The product is obtained in a freeze-dried form as a white and flocculent low-density powder. The purity of said product is at least 98 %. It may be drawn off in amounts of from 10 to 500 µg and be dissolved prior to use in physiological saline (0.9% of NaCl). For example, the substance is drawn off in amounts of 50 µg as a lyophilized powder without auxiliary materials in 2 ml glass vials. 1 ml of physiological saline (0.9% of NaCl) was used for dissolution. These solutions may be perfectly injected or infused. Basically there is also the possibility to prepare and store the peptide in a physiological saline or any other isotonic physiologically compatible solution. Optionally a physiologically compatible disinfectant and/or stabilizer will be added to these solutions.

In order to determine the respective fractions containing the desired biologically active cardiodilatin fragment in chromatographic separations, per se known methods for detecting the biological activity are employed. For the detection of urodilatin (ANF/CDD 95-126) or of ANF/CDD 99-126 in one fraction, there is suitable the relaxing effect of the compounds on the smooth musculature. In this assay, it is preferred to use the Arteria renalis of rabbits, the Aorta abdominalis of rabbit or rat or the Arteria pulmonalis of rabbits in the organ bath, where the stripes of vessel muscles contracted upon a norepinephrine pretreatment show a significant relaaxation after the addition of the active cardiodilatin fragment. This reaction is best recognizable in muscle stripes from Arteria renalis, with which the relaxation is detectable already at doses of about from 1.0 to 10.00 mg per 10 ml in the organ bath.

It appears that the disulfide bridge as contained between the amino acids Cys 105 and Cys 121 is essential for the biological action.

These biological actions show that the peptide hormone cardiodilatin, and surprisingly the fragment urodilatin (ANF/CDD 95-126) as well, have a great clinical, diagnostic and therapeutic importance. More specifically, the cardiodilatin fragment urodilatin (ANF/CDD 95-126) is suitable for the following fields of application: differentiated vasodilation of certain vessel beds, diagnostics and therapy of hypertonia, application as a substitution for patients, which have been implanted with artificial hearts, synchronous regulation of blood volume and electrolytes of the blood, skin diseases, and more particularly those including perspiration disorders, cardiovascular shock, renal and adrenal cortical disorders, diseases of the gastro-intestinal tract, and more particularly motility disorders and obstipation, therapy of brain edemae and of glaucoma, therapy of spastic coronaropathies such as angina pectoris, for the treatment of acute renal insuffiency, nephrotic and nephritic syndrome, terminal renal insufficiency, acute coronary insufficiency, ascites, generalized edemae, lung edema, brain edema, primary and secondary lymph edemae, hydrothorax, glaucoma, vena cava stenosis, hypoproteinemia, hearing impairment, essential and renal hypertonia, malignant hypertonia, EPH gestosis such as hypertonia of pregnancy, nephrosis of pregnancy, cerebral sodium storage syndrome, vessel spasms such as Morbus Raynaud, angina abdominalis, coronary spasms, coronary heart disease, vasomotor headache, headache upon hypertonia, Bing-Horton syndrome migraine, blood circulation disorders in the vertebralis-basilaris flow path region, disorders of the renin-angiotensin system, primary and secondary hyperaldosteronism, renin-secernent tumors, phaeochromocytoma, Bartter's syndrome, Schwartz-Bartter's syndrome, pancreas insufficiency, primary sweat gland insufficiency such as anhidrosis, miction and defecation disorders, dysregulation of the anterior lobe of the pituitary gland, cardiovascular side-effects in the therapy Of psych(iatr)ic diseases and agitation with catechol increase, for the supporting treatment upon heart transplantations, "PEEP-respiration" (an artificial respiration with positive end expiratory pressure) as well as by-pass operations and for the treatment of complications after implantation of an artificial heart. Urodilatin (ANF/CDD 95-126) may also be employed as a diuretic for the treatment of multiple diseases such as gout combined with hypertonia/heart insufficiency, as well as diabetes with hypertonia/heart insufficiency, or as a diuretic in combination with cephalosporins, aminoglycosides or anticoagulants. Urodilatin (ANF/CDD 95-126) is further suitable for the prophylaxis of an acute renal failure after kidney transplantations and treatment with nephrotoxic substances such as cyclosporin, cisplatin and ifosfamid and for the prophylaxis of an acute kidney failure which may occur postoperative or after hypertonic crisis as well as after dosis of contrast media for patients suffering from limited functions of the kidney. The agent according to the invention is also usable as a diagnostic for differential diagnostics of endothelial changes.

Therefore, subject matter of the invention is also medicaments containing the new cardiodilatin fragment urodilatin (ANF/CDD 95-126) for use in the diagnosis and therapy methods as set forth above. The medicaments may be present in the conventional application forms for intravenous, oral or parenteral administrations such as, e.g., as tablets, suppositories, dragées, solutions, sprays or preparations for inhalation etc., as well as micro-encapsulation, if desired in combination with conventional pharmacological excipients and/or diluents. The medicament according to the invention can also be formulated as slow-release-form. Preferably the amount of urodilatin (ANF/CDD 95-126) is from 50 ng to 50 µg per unit dose.

Furthermore, according to the invention there is provided a method for the specific determination of cardiodilatin which is based on the principle of immunoassay and employs an antibody directed against urodilatin (ANF/CDD 95-126). Surprisingly it has been determined that by using antibodies directed against urodilatin (ANF/CDD 95-126) cardiodilatin can be highly specifically and very accurately detected in body liquids. Said method for the specific determination of cardiodilatin can be used for the diagnosis of neurological diseases by specifically determining cardiodilatin or is fragments in the liquor cerebrospinalis.

The use of the medicament according to the invention which contains, as the active substance, urodilatin (ANF/CDD 95-126), due to the beneficial spectrum of actions and low toxicity thereof, is indicated for the treatment of diseases involving disorders of the electrolyte and water balance such as, for example, pre-terminal and terminal renal insufficiency, malfunctions of the renin-angiotensin-aldosteron system, disorders of the vasopressin secretion, at liquid sequestration ("Third Space" problems such as ascites, glaucoma, brain edema, hydrothorax) as well as other diseases involving an increased extravasal volume accumulation such as lymph edema, EPH gestosis, vena cava stenosis, diseases involving hypoproteinemia such as renal diseases, enteropathioes and liver diseases.

The medicament according to the invention, due to its vasodilative properties, may be used for the treatment of all diseases which are accompanied by an increased vasoconstriction. Thus, the medicament containing urodilatin (ANF/CDD 95-126) according to the invention may be used for the treatment of spasms of vessels such as, for example, Morbus Raynaud, spasms of the muscular distributor arteriae and hearing impairment. Urodilatin (ANF/CDD 95-126) may also be employed for treating further vascular diseases such as, for example., for the treatment of coronary heart disease, Angina abdominalis, vasomotor headache, Arteria basilaris migraine with blood circulation disorders in the vertebralis-basilaris flow path region and Bing-Horton syndrome.

The medicament containing urodilatin (ANF/CDD 95-126) according to the invention, due to its blood pressure-lowering properties which become effective above all with primarily increased blood pressure, is indicated for malignant hypertonia, hypertonia of pregnancy, headache upon hypertonia, hypertonia in combination with other diseases, for example gout and diabetes.

The medicament according to the invention exhibits antagonistic properties against sodium-retaining, water-retaining and vasoconstrictive hormones. This enables the use of the medicament according to the invention for the treatment of primary and secondary hyperaldosteronism, renovascular hypertony, renin-secernant tumors, phaeochromocytoma, Bartter's syndrome and Schwartz-Bartter's syndrome.

The medicament containing urodilatin (ANF/CDD 95-126) according to the invention can further be used for the treatment of heart insufficiency, in heart transplantations and with artificial hearts, for support in "PEEP" (positive end expiratory pressure), during by-pass operations and with open heart surgery. In respiration with positive end expiratory pressure expiration is effected against superatmospheric pressure.

The medicament according to the invention can be used for prophylaxis of acute renal failure after kidney transplantations, postoperatively and for improving the function of transplantate kidneys by pretreating the transplantate kidneys with the medicament of the invention.

The medicament containing urodilatin (ANF/CDD 95-126) according to the invention can also be used for diagnostic purposes. Since the vasodilation is endothel-independent, a comparison with an endothel-dependent vasodilator allows to make a statement on the condition of the endothel.

The use of the medicament according to the medicament according to the invention is also indicated at gastro-intestinal indications such as changes in the pancreas secretion and concomitant utilization disorders, nutritional defects, exocrine pancreas insufficiency, regulations of bowels and bladder (miction and defecation disorders).

In dermatology, the medicament according to the invention may be used for combatting anhidrosis. Also in psychiatry the agent according to the invention may be used for the treatment of cardiovascular side-effects in the therapy of psych(iatr)ic diseases and agitation with catechol release.

These effects are observed already at concentrations within the nanomolar range. However, due to the low toxicity and good compatibility of the agent, higher concentrations may be injected or infused as well.

Urodilatin (ANF/CDD 95-126), in the highly pure form as obtainable by solid phase synthesis, may be used for the preparation of medicaments such as solutions for injection or infusion, nasal spray, ophthalmic drops or as a slow-release form in the case of indications as mentioned above.

The examination of acute toxicity to animals has shown that in a dose range up to 400 µg/kg of body weight no toxic reactions do occur which are detectable using conventional control parameters (biochemistry). An LD₅₀ value is not determinable because of the low toxicity of the substance. No serious side-effects were observed in the heart-circulation system, bronchial system, liver and kidney function, gonads and central nervous system.

The pharmacological efficiency of a treatment of the above mentioned diseases ensues from the observed sodiuretic effect which upon intravenous administration results in a rapid increase in diuresis and sodium excretion. Furthermore it has been observed that urodilatin (ANF/CDD 95-126) is capable of compensating the vasoconstrictive effet of noradrenalin.

It has further been observed that the substance inhibits the contracting effect of angiotensin. Of particular interest are cases of serious heart insufficiency with generalized edematosis which do not respond to a conventional therapy. Of considerable importance is also the action on the blood pressure-controlling systems and the vasopressin-controlling systems, so that functional disorders of the posterior lobe of the pituitary gland and psychotropic effects caused thereby are indicated. It is not to be excluded that a more intensive clinical investigation of this substance will result in that further interesting indications are fould.

The invention is further illustrated by the following FIGURES and EXAMPLES:

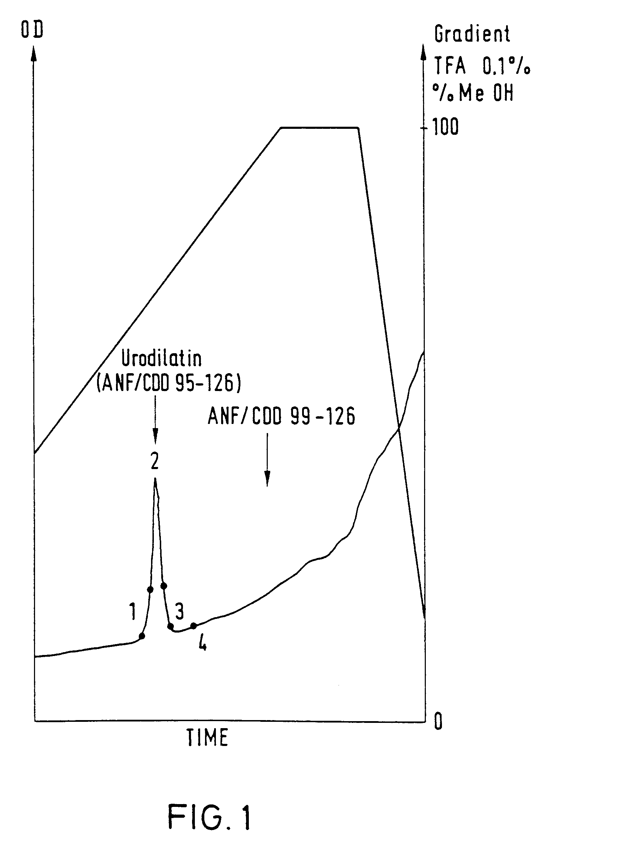

- Fig. 1: shows a HPLC chromatogram of urodilatin (ANF/CDD 95-126), isolated from urine. The eluant is methanol.
- Rf Value:: 25 minutes
- Conditions::
- Column:: TSK-ODS-120T, 250 mm x 4.8 mm
- Mobile Solvent A:: 40% methanol in water (0.1% of trifluoroacetic acid)
- Mobile Solvent B:: 100% methanol (of 0.1% trifluoroacetic acid)
- Gradient:: 10 minutes isocratically A, then during addition of B during 30 minutes increasing to 100% of B
- Flow Rate:: 0.5 ml/min;
20 °C; paper feed 5 mm/min; calculated absorbance 0.08, UV 254 nm.
- Fig. 2: shows a HPLC chromatogram of ANF/CDD 99-126 using methanol as eluant for comparison. Rf Value: 35 minutes. The conditions were the same as in Fig. 1.

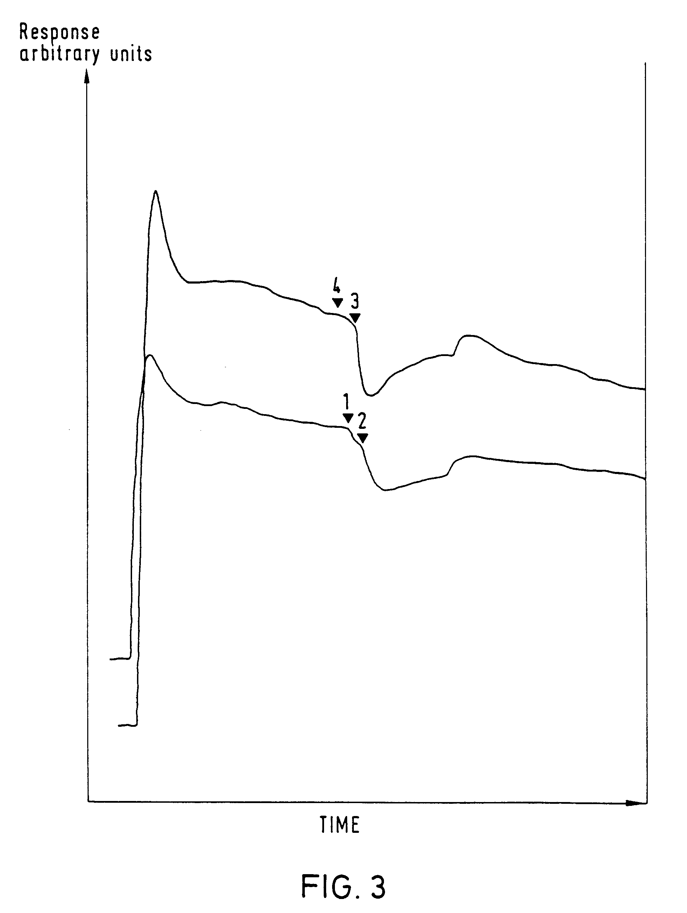

- Fig. 3: shows a diagram, wherein biological activity of urodilatin (ANF/CDD 95-126) is demonstrated. Tested were the fractions 1 to 4 of the HPLC as shown in Fig. 1 in the relaxation assay using aorta muscle strips. The doses were 1 µl of each of the HPLC fractions per 10 ml of organ bath {1 µl corresponds to about 3 µg of urodilatin (ANF/CDD 95-126)}.

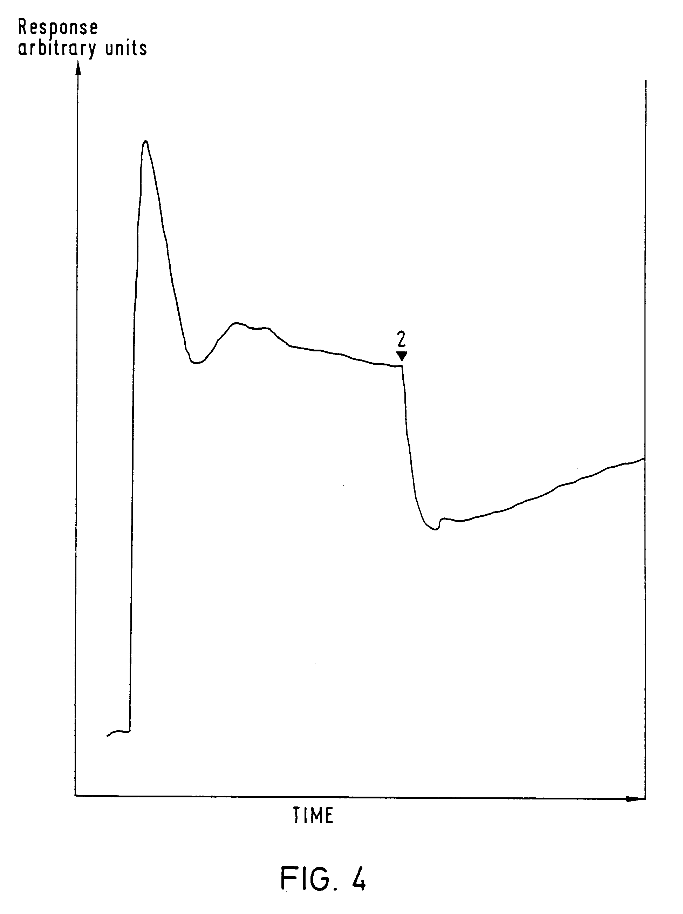

- Fig. 4: shows a diagram, wherein the biological activity of the fraction II is demonstrated in the same manner as in Fig. 3.
- Fig. 5: shows a HPLC diagram. The hatched area represents the main proportion having bioactivity of the cardiodilatin type.
- Fig. 6: shows a HPLC diagram comprising 10 individual peaks, only one of which, i.e. the peak of a retention time of 28.2 min (hatched) contains the biologically active substance.

### EXAMPLE 1

Urine of healthy humans was employed. The urine was immediately acidified with glacial acetic acid to a final concentration of 0.2M. Batches of 200 to 400 l each were diluted with water in a ratio of 1:1 and then adjusted to a pH 2.7 with concentrated hydrochloric acid. Added to the first batch were 2.5 kg of alginic acid, and the mixture was stirred from 8 to 12 hours. Then the alginic acid settled, and the supernatant was replaced by a new batch of acidified urine. This procedure was repeated until more than 1,000 l of urine had been reacted with the 2.5 kg of alginic acid. Then the alginic acid was sedimented, separated from the supernatant and washed with ethanol and 0.005M hydrochloric acid on a Büchner funnel. The polypeptides precipitated on the alginic acid were then eluted with 0.2M hydrochloric acid. The pH of the eluate was adjusted to 4.0, and sodium chloride was added to saturation. After 24 hours at 4 °C, a salt cake was taken off which had been formed on the salt-saturated solution.

This procedure was monitored by means of an radio-immunoassay for cardiodilatin whereby it showed that more than 90% of the detectable substance had been obtained. Then the salt cake was charged onto a Sephadex G-25 column, and the peptides obtained were tested by means of a bioassay for cardiodilatin using aorta muscle stripes. The fraction containing most of the cardiodilatin bioactivity were then lyophilized and chromatographed on an ion exchanger column. The bioactive vasorelaxant material was found in those fractions which were eluted at the highest ionic strength with stepwise gradient.

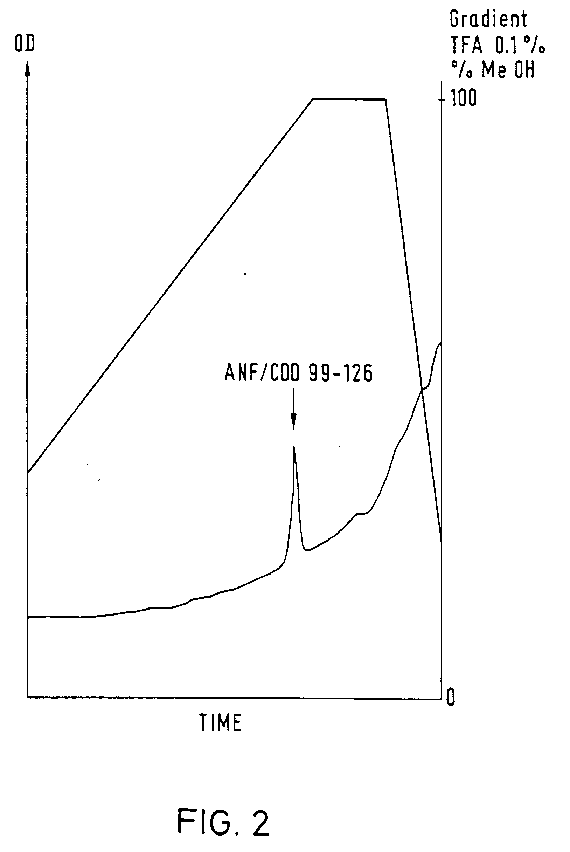

In the first HPLC there was used a reversed-phase column TSK-ODS-120T. As the eluant there was employed water-acetonitrile-HCl with a continuous gradient. The bioactive materials appears at the same location where syntehtic ANF/CDD 99-126 as employed for comparison is eluted. The bioactive fractions were charged onto an analytical reversed-phase column of the same type, while elution was effected using water-methanol-trifluoroacetic acid with a continuous gradient as shown in Fig. 1. Now the bioactive material exhibited a retention time of 25 minutes, whereas synthetic ANF/CDD 99-126 as employed for comparison was eluted after 35 minutes (Fig. 2).

### EXAMPLE 2

### Structure Elucidation:

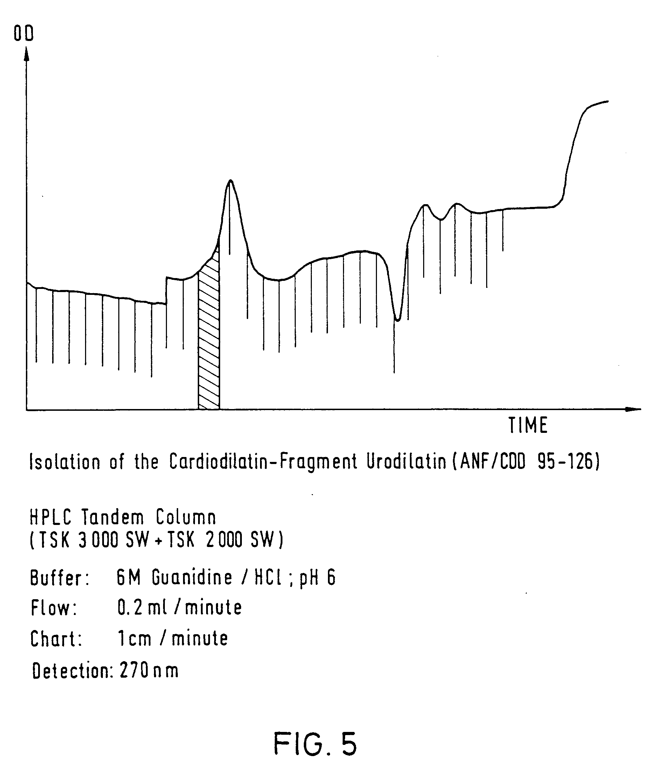

The biological material accumulated from human urine according to Fig. 1 and having cardiodilatin-like activity other than ANF/CDD 99-126 (Fig. 2) was subjected to chromatography on a HPLC tandem column (TSK 3000 SW + TSK 2000 SW) in the buffer system 6M guanidine/HCl (50 mM PO₄) at pH 6.0 and room temperature. The major proportion of the cardiodilatin-like bioactivity was found in the minor fraction 62 of this purification step (hatched area in Fig. 5). The liquid volume of the fraction 62 was adjusted to 0.1% with trifluoroacetic acid and applied onto a C-18-SEPAK (tradename) cartridge dor desalting. To this end the loaded cartridge was subsequently washed with water (0.1% of trifluoroacetic acid) until salt-free. Then the biologically active material was eluted from the cartridge with methanol (or acetonitrile). The eluate was lyophilized. The lyophilizate was again taken up in water (0.1% of trifluoroacetic acid) and separated by means of RP-HPLC.
- Column:: 125 mm x 4 mm; C-4-phase, ORPEGEN, HD gel.
- Buffer A:: Water (0.1% of trifluoroacetic acid)
- Buffer B:: 20% of water in acetonitrile (of 0.1% trifluoroacetic acid)
- Flow Rate:: 0.5 ml/min;
- Temperature:: 45 °C
- Detection:: 230 nm/0.04 Det.Sens.

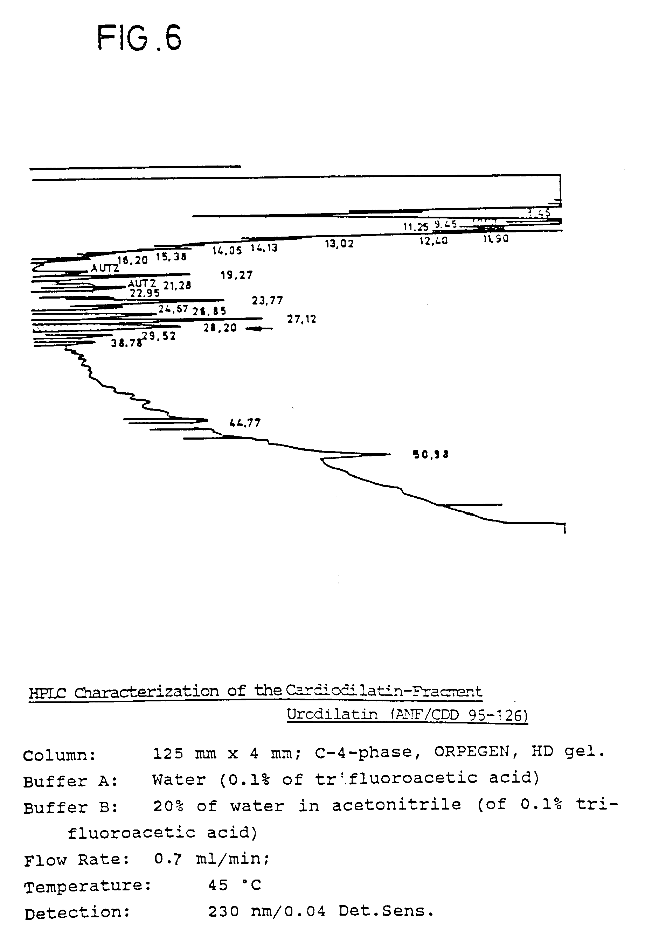

The HPLC diagram showed more than ten individual peaks in the separation field (Fig. 6), only one of which (marked by an arrow) at a retention time of 28.2 minutes contains the biologically active substance. This peak has been designated as Peak No. 8.

### Sequence Analysis:

A stepwise Edmann degradation of the intact peptide (Peak No. 8, retention time 28.2 minutes in Fig. 6) was carried out in a gas phase-protein-sequencer 420 A of the company Applied Biosystems. The PTH amino acids were identified by high performance liquid chromatography according to Lottspeich ("High Performance Liquid Chromatography in Protein and Peptide Chemistry", pages 259-268, Lottspeich F., Henschen A., Hupe K.P.; Walter de Gryter, Berlin/New York 1981). The highly purified polypeptide had the following amino acid sequence:

Thr-Ala-Pro-Arg-Ser-Leu-Arg-Arg-Ser-Ser-Cys-Phe-Gly-
Gly-Arg-Met-Asp-Arg-Ile-Gly-Ala-Gln-Ser-Gly-Leu-Gly-
Cys-Asn-Ser-Phe-Arg-Tyr-OH.

### Amino Acid Analysis:

The highly purified polypeptide (Peak No. 8, retention time 28.2 minutes in Fig. 6) was hydrolyzed in 6M HCl, 1.0% of phenol at 110 °C in evacuated tubes for 24 hours. The total composition was determined in a Waters-Amino Acid-Analysis System after derivatization with phenylisothiocyanate. The analysis showed a high degree of conformity between the primary structure as obtained by sequence analysis and the total composition, and more specifically so for the values of Tyr 0.9 (1); Met 0.99 (1); Cys 2.1 (2); Ile 1.1 (1); Leu 2.1 (2); Arg 6.4 (6).

### EXAMPLE 3

### Stepwise Synthesis on a Solid Phase (ABI-Synthesizer 430)

Starting with 1.22 g of Boc-Tyr (Bzl) -Merrifield support (0.67 mmol/g; 1% of DVB; 200 - 400 mesh) the sequence is synthesized according to the ABI Standard program according to the Boc strategy. Double couplings are carried out up to Ile (113), and triple couplings are carried out from Arg (112). The following Boc amino acids are employed:

Boc-Gly, Boc-Ala, Boc-Leu, Boc-Ile, Boc-Phe, Boc-Met,
Boc-Pro, Boc-Asn, Boc-Gln, Boc-Asp (OBzl), Boc-Ser
(Bzl), Boc-Thr (Bzl), Boc-Cys (Acm), Boc-Arg (Tos).

The dried peptide-polystyrene is treated with HF with the addition of 5% of anisol and 2.5% of ethylmethylsulfide at 0 °C for 1 hour. Upon removal of the HF in vacuo the crude peptide is washed with AcOH from the polymeric support, and the filtrates are lyophilized. Then the crude lyophilizate is desalted on Sephadex LH 20/water (1% AcOH/1% TFEtOH).

For Acm-removal and cyclization a 5 mM solution of the peptide AcOH/H₂0 (9:1; v:v) is prepared, and 1.5 eq. of HCl is added thereto. A concentrated solution of 10 eq. of iodine in AcOH are added at once with vigorous stirring to the peptide solution. After 15 minutes the reaction is terminated by the addition of a diluted ascorbic acid solution in 0.5M citric acid. After lyophilization the material is desalte over Sephadex LH 20/water (1% AcOH/1% TFEtOH) and purified after re-chromatography on Fractogel TSK-HW 40/water (10% AcOH/1% TFEtOH) by means of preparative HPLC.

### EXAMPLE 4

### Synthesis by Fragment Condensation on a Polymeric Support

On the analogy of Example I the sequence is built up up to Ser (99). After Boc-removal and deprotonation of 0.5 g of peptide (99-126) -polystyrene coupling is effected in 5 ml of dichloromethane in a shaker reactor at 20 °C for 72 hours by means of 1.609 g (2.1775 mMol; 5-fold excess) of Boc-Thr(But)-Ala-Pro-Arg(Tos) which had been pre-activated with 0.353 g (2.1775 mMol) of 1,1'-carbonyldiimidazole/0.667 g (4.36 mMol) of 1-hydroxybenzotriazole in 5 ml of dichlormethane for 30 minutes. The detachment from the support, Acm-removal, cyclization and work-up and purification are carried out on the analogy of Example I.

### EXAMPLE 5

### Synthese in Solution by Fragment Condensation

2.01 mg (2.72 µMol) of Boc-Thr(But)-Ala-Pro-Arg(Tos) are pre-activated with 0.44 mg (2.72 µMol) of 1,1'-carbonyldiimidazol/0.83 mg (5.44 µMol) of 1-hydroxybenzotriazole in 2 ml of DMF for 30 minutes and added to a solution of 5 mg (1.36 µMol; peptide content: 84%) of ANF/CDD 99-126 and 0.15 µl (1.36 µMol) of N-Methylmorpholine in 3 ml of DMF. After stirring at 20 °C for 72 hours the mixture is concentrated in vacuo, admixed with a small amount of a NaHCO₃ solution, then diluted with a small amount of 1% AcOH/1% TFEtOH and chromatographed on LH 20 in 1% AcOH/1% TFEtOH. After treatment with HF/5% anisol/2.5% ethylmethylsulfide the material is subjected to chromatography on LH 20/water (1% AcOH/1% TFEtOH) and then on Fractogel TSK-HW 40 (S)/water (10% AcOH/1% TFEtOH) and finally purified by means of preparative HPLC.

## Claims

1. A peptide having the amino acid sequence 95-126 of ANF/CDD 1-126 (gamma-hANaP) having the designation urodilatin (ANF/CDD 95-126) according to the formula

Thr-Ala-Pro-Arg-Ser-Leu-Arg-Arg-Ser-Ser-Cys-Phe-Gly-Gly-Arg-Met-
|
S
|
S
|
Asp-Arg-Ile-Gly-Ala-Gln-Ser-Gly-Leu-Gly-Cys-Asn-Ser-Phe-Arg-Tyr

2. A process for preparing the cardiodilatin fragment urodilatin (ANF/CDD 95-126) according to claim 1 by stepwise synthesis on a solid phase using amino acids protected by protective groups.

3. The process according to claim 2, characterized in that tert-butyloxycarbonyl, fluorenylmethyloxycarbonyl and/or 3,5-dimethoxyphenyl-2,2-propyloxycarbonyl groups are used as protective groups.

4. A process for preparing the cardiodilatin fragment urodilatin (ANF/CDD 95-126) according to claim 1, characterized in that ANF/CDD 99-126 which has been bonded to a solid phase is reacted with the tetrapeptide Thr-Ala-Pro-Arg, the resulting peptide is detached from the carrier, subjected to cyclization after removal of the protective groups and worked up and purified in a per se known manner.

5. A process for preparing the cardiodilatin fragment urodilatin (ANF/CDD 95-126) according to claim 1, characterized in that the peptide ANF/CDD 99-126 is dissolved in a solvent, the tetrapeptide Thr-Ala-Pro-Arg, provided with protective groups, if appropriate, is added together with an activating agent, the resulting product is subjected to chromatography after removal of the protective groups is further purified in a per se known manner.

6. The process according to anyone of claims 4 or 5, characterized in that Boc-Thr(But)-Ala-Pro-Arg(Tos) is employed as the tetrapeptide.

7. A process for recovering urodilatin (ANF/CDD 95-126) according to claim 1 from body liquids, characterized in that urine is used as the starting material, alginic acid is added to the solution, the peptides adsorbed on the alginic acid are eluted, the eluate is fractionated according to conventional biochemical purification methods, and the active fraction is determined by conventional methods for the detection of the biological activity.

8. The process according to claim 7, characterized in that the urodilatin (ANF/CDD 95-126) adsorbed on the alginic acid is eluted, the eluate is subjected to a salt precipitation or is lyophilized, the precipitate is desalted by gel chromatography, the obtained crude extract is subjected to ion exchange chromatography and subsequently separated by HPLC.

9. The process according to claim 8, characterized in that the separation by HPLC is carried out using a reversed-phase silicagel.

10. A process for recovering urodilatin (ANF/CDD 95-126), characterized in that urine is contacted with alginic acid, the urodilatin (ANF/CDD 95-126) adsorbed on the alginic acid is eluted, the eluate is fractionated according to conventional biochemical purification methods using a test in which the fraction containing urodilatin (ANF/CDD 95-126) is determined by way of its relaxing effect on the smooth musculature.

11. Medicament, containing urodilatin (ANF/CDD 95-126) according to claim 1 as the active ingredient in combination with a pharmacologically compatible carrier and/or diluent.

12. The medicament according to claim 11 for diagnostics and therapy of hypertonia, vessel and heart diseases, skin diseases including perspiration disorders, cardiovascular shock, renal and adrenal cortical disorders, diseases of the gastro-intestinal tract, and more particularly motility disorders, brain edemae, glaucoma, spastic coronaropathies and neurological diseases.

13. The medicament according to claim 11 for the treatment of acute renal insuffiency, nephrotic and nephritic syndrome, terminal renal insufficiency, acute coronary insufficiency, ascites, generalized edemae, lung edema, brain edema, primary and secondary lymph edemae, hydrothorax, glaucoma, vena cava stenosis, hypoproteinemia, hearing impairment, essential and renal hypertonia, malignant hypertonia, EPH gestosis such as hypertonia of pregnancy, nephrosis of pregnancy, cerebral sodium storage syndrome, vessel spasms such as Morbus Raynaud, angina abdominalis, coronary spasms, coronary heart disease, vasomotor headache, headache upon hypertonia, Bing-Horton syndrome migraine, blood circulation disorders in the vertebralis-basilaris flow path region, disorders of the renin-angiotensin system, primary and secondary hyperaldosteronism, renin-secernent tumors, phaeochromocytoma, Bartter's syndrome, Schwartz-Bartter's syndrome, pancreas insufficiency, primary sweat gland insufficiency such as anhidrosis, miction and defecation disorders, dysregulation of the anterior lobe of the pituitary gland, cardiovascular side-effects in the therapy of psych(iatr)ic diseases and agitation with catechol increase.

14. The medicament according to claim 11 for the supporting treatment upon heart transplantations, "PEEP-respiration" (an artificial respiration with positive end expiratory pressure) as well as by-pass operations.

15. The medicament according to claim 11 for the treatment of complications after implantation of an artificial heart.

16. The medicament according to claim 11 as a diuretic for the treatment of multiple diseases such as gout combined with hypertonia/heart insufficiency, as well as diabetes with hypertonia/heart insufficiency.

17. The medicament according to claim 11 as a diuretic in combination with cephalosporins, amino-glycosides or anticoagulants.

18. The medicament according to claim 11 for the prophylaxis of an acute renal failure after kidney transplantations and treatment with nephrotoxic substances such as cyclosporin, cisplatin and/or ifosfamid.

19. The medicament according to claim 11 for the prophylaxis of an acute kidney failure which may occur postoperative or after hypertonic crisis as well as after dosis of contrast medium for a patient suffering from limited function of the kidney.

20. The medicament according to claim 11 as a diagnostic for differential diagnostics of endothelial changes.

21. The medicament according to claim 11, characterized in that it contains from 10 ng to 50 µg of urodilatin (ANF/CDD 95-126) per dosage unit.

22. The medicament according to claim 11 having a vasodilative effect, characterized in that it contains urodilatin (ANF/CDD 95-126) as the active ingredient.

23. A method for the specific determination of cardiodilatin, characterized in that it works according to the principle of immunoassay and employs an antibody directed against urodilatin (ANF/CDD 95-126).

24. Use of the urodilatin (ANF/CDD 95-126) according to claim 1 for the preparation of a diagnostic for differential diagnostics of endothelial changes.

25. Use of the urodilatin (ANF/CDD 95-126) according to claim 1 for the preparation of medicaments such as injection or infusion solutions for the treatment of acute renal insuffiency, nephrotic and nephritic syndrome, terminal renal insufficiency, acute coronary insufficiency, ascites, generalized edemae, lung edema, brain edema, primary and secondary lymph edemae, hydrothorax, glaucoma, vena cava stenosis, hypoproteinemia, hearing impairment, essential and renal hypertonia, malignant hypertonia, EPH gestosis such as hypertonia of pregnancy, nephrosis of pregnancy, cerebral sodium storage syndrome, vessel spasms such as Morbus Raynaud, angina abdominalis, coronary spasms, coronary heart disease, vasomotor headache, headache upon hypertonia, Bing-Horton syndrome migraine, blood circulation disorders in the vertebralis-basilaris flow path region, disorders of the renin-angiotensin system, primary and secondary hyperaldosteronism, renin-secernent tumors, phaeochromocytoma, Bartter's syndrome, Schwartz-Bartter's syndrome, pancreas insufficiency, primary sweat gland insufficiency such as anhidrosis, miction and defecation disorders, dysregulation of the anterior lobe of the pituitary gland, cardiovascular side-effects in the therapy of psych(iatr)ic diseases and agitation with catechol increase.

26. Use of the urodilatin (ANF/CDD 95-126) according to claim 1 for the preparation of medicaments such as injection or infusion solutions for the supporting treatment upon heart transplantations, "PEEP-respiration" (an artificial respiration with positive end expiratory pressure) as well as by-pass operations.

27. Use of the urodilatin (ANF/CDD 95-126) according to claim 1 for the preparation of medicaments such as injection or infusion solutions for the treatment of complications after implantation of an artificial heart.

28. Use of the urodilatin (ANF/CDD 95-126) according to claim 1 for the preparation of medicaments such as injection or infusion solutions as a diuretic for the treatment of multiple diseases such as gout combined with hypertonia/heart insufficiency, as well as diabetes with hypertonia/heart insufficiency.

29. Use of the urodilatin (ANF/CDD 95-126) according to claim 1 for the preparation of medicaments such as injection or infusion solutions as a diuretic in combination with cephalosporins, aminoglycosides or anticoagulants.

30. Use of the urodilatin (ANF/CDD 95-126) according to claim 1 for the preparation of medicaments such as injection or infusion solutions for the prophylaxis of an acute renal failure after kidney transplantations and treatment with nephrotoxic substances such as cyclosporin, cisplatin and/or ifosfamid.

31. Use of the urodilatin (ANF/CDD 95-126) according to claim 1 for the preparation of medicaments such as injection or infusion solutions for the prophylaxis of an acute kidney failure which may occur postoperative or after hypertonic crisis as well as after dosis of contrast medium for a patient suffering from limited function of the kidney.

32. Use of the urodilatin (ANF/CDD 95-126) according to claim 1 for the preparation of medicaments such as injection or infusion solutions as a diagnostic for differential diagnostics of endothelial changes.

## Patentansprüche

1. Peptid mit der Aminosäuresequenz 95-126 des ANF/CDD 1-126 (Gamma-hANaP) mit der Bezeichnung Urodilatin (ANF/CDD 95-126) gemäß der Formel:

Thr-Ala-Pro-Arg-Ser-Leu-Arg-Arg-Ser-Ser-Cys-Phe-Gly-Gly-Arg-Met-
S
S
Asp-Arg-Ile-Gly-Ala-Gln-Ser-Gly-Leu-Gly-Cys-Asn-Ser-Phe-Arg-Tyr.

2. Verfahren zur Herstellung des Cardiodilatinfragmentes Urodilatin (ANF/CDD 95-126) nach Anspruch 1 durch schrittweisen Aufbau an fester Phase unter Verwendung von mit Schutzgruppen geschützten Aminosäuren.

3. Verfahren nach Anspruch 2, dadurch gekennzeichnet, daß als Schutzgruppen tert.-Butyloxycarbonyl-, Fluorenylmethyloxycarbonyl- und/oder 3,5-Dimethoxyphenyl-2,2-propyloxycarbonylgruppen verwendet werden.

4. Verfahren zur Herstellung des Cardiodilatinfragmentes Urodilatin (ANF/CDD 95-126) nach Anspruch 1, dadurch gekennzeichnet, daß man ANF/CDD 99-126, das an eine Festphase gebunden vorliegt, mit dem Tetrapeptid Thr-Ala-Pro-Arg umsetzt, das entstandene Peptid vom Träger abspaltet, nach Abspaltung der Schutzgruppen cyclisiert sowie in an sich bekannter Weise aufarbeitet und reinigt.

5. Verfahren zur Herstellung des Cardiodilatinfragmentes Urodilatin (ANF/CDD 95-126) nach Anspruch 1, dadurch gekennzeichnet, daß man das Peptid ANF/CDD 99-126 in einem Lösungsmittel auflöst, das gegebenenfalls mit Schutzgruppen versehene Tetrapeptid Thr-Ala-Pro-Arg zusammen mit Aktivierungsmittel Zugibt, nach Abspaltung der Schutzgruppen chromatographiert und in an sich bekannter Weise weiter reinigt.

6. Verfahren nach einem der Ansprüche 4 oder 5, dadurch gekennzeichnet, daß man als Tetrapeptid Boc-Thr(But)-Ala-Pro-Arg(Tos) einsetzt.

7. Verfahren zur Gewinnung von Urodilatin (ANF/CDD 95-126) nach Anspruch 1 aus Körperflüssigkeiten, dadurch gekennzeichnet, daß man Urin als Ausgangsmaterial verwendet, der Lösung Alginsäure zusetzt, die an der Alginsäure adsorbierten Peptide eluiert und das Eluat nach üblichen biochemischen Reinigungsmethoden fraktioniert und die aktive Fraktion durch übliche Methoden zum Nachweis der biologischen Aktivität bestimmt.

8. Verfahren nach Anspruch 7, dadurch gekennzeichnet, daß man das an der Alginsäure adsorbierte Urodilatin (ANF/CDD 95-126) eluiert, das Eluat einer Salzfällung unterwirft oder lyophylisiert, den Niederschlag mittels Gelchromatographie entsalzt, den erhaltenen Rohextrakt einer Ionenaustausch-Chromatographie unterwirft und anschließend über HPLC auftrennt.

9. Verfahren nach Anspruch 8, dadurch gekennzeichnet, daß man die Auftrennung über HPLC an einem Umkehrphasen-Kieselgel durchführt.

10. Verfahren zur Gewinnung von Urodilatin (ANF/CDD 95-126), dadurch gekennzeichnet, daß man Urin mit Alginsäure in Berührung bringt, das an der Alginsäure adsorbierte Urodilatin (ANF/CDD 95-126) eluiert und das Eluat nach üblichen biochemischen Reinigungsmethoden fraktioniert unter Anwendung eines Tests, bei dem die Fraktion, die Urodilatin (ANF/CDD 95-126) enthält, durch die relaxierende Wirkung auf die glatte Muskulatur bestimmt wird.

11. Arzneimittel enthaltend als Wirkstoff Urodilatin (ANF/CDD 95-126) gemäß Anspruch 1 zusammen mit einem pharmakologisch verträglichen Träger und/oder Verdünnungsmittel.

12. Arzneimittel nach Anspruch 11 zur Diagnose und Therapie von Hypertonie, Gefäß- und Herzkrankheiten, Hauterkrankungen mit Störungen der Schweißsekretion, des cardiovaskulären Schocks, von Erkrankungen der Nieren und Nebennierenrinde, Erkrankungen des Gastrointestinaltraktes, insbesondere Motilitätsstörungen, des Hirnödems, des Glaukoms, spastischer Coronaropathien sowie von neurologischen Erkrankungen.

13. Arzneimittel nach Anspruch 11 zur Behandlung von akuter Niereninsuffizienz, nephrotischem und nephritischem Syndrom, terminaler Niereninsuffizienz, akuter Herzinsuffizienz, Ascites, generalisierten Ödemen, Lungenödem, Hirnödem, primären und sekundären Lymphödemen, Hydrothorax, Glaukom, Vena-cava-Stenose, Hypoproteinämie, Hörsturz, essentieller und renaler Hypertonie, maligner Hypertonie, EPH-Gestose wie Schwangerschaftshypertonie, Schwangerschafts-Nephrose, zerebralem Natriumspeichersyndrom, Gefäßspasmen wie Morbus Raynaud, Angina abdominalis, Koronarspasmen, koronarer Herzkrankheit, vasomotorischen Kopfschmerzen, Kopfschmerzen bei Hypertonie, Bing-Horton-Syndrom Migräne, Durchblutungsstörungen im Vertebralis-Basilaris-Strombahngebiet, Störungen des Renin-Angiotensin-Systems, primärem und sekundärem Hyperaldosteronismus, reninsezernierenden Tumoren, Phäochromozytom, Bartters Syndrom, Schwartz-Bartter-Syndrom, Pankreasinsuffizienz, primärer Schweißdrüseninsuffizienz wie Anhidrosis, Miktions- und Defäkationsstörungen, Hypophysenvorderlappendysregulation, cardio-vaskulären Nebenwirkungen bei der Therapie psychiatrischer Erkrankungen sowie Agitation mit Catecholaminerhöhung.

14. Arzneimittel nach Anspruch 11 zur unterstützenden Behandlung bei Herztransplantationen, "PEEP-Beatmung" (einer mechanischen Beatmung mit positivem endexpiratorischem Druck) sowie Bypass-Operationen.

15. Arzneimittel nach Anspruch 11 zur Behandlung von Komplikationen nach Einpflanzung eines künstlichen Herzens.

16. Arzneimittel nach Anspruch 11 als Diuretikum zur Behandlung von multiplen Erkrankungen wie Gicht, kombiniert mit Hypertonus/Herzinsuffizienz sowie Diabetes mit Hypertonus/Herzinsuffizienz.

17. Arzneimittel nach Anspruch 11 als Diuretikum in Kombination mit Cephalosporinen, Aminoglykosiden oder Antikoagulantien.

18. Arzneimittel nach Anspruch 11 zur Prophylaxe von akutem Nierenversagen nach Nierentransplantationen und Behandlung mit nephrotoxischen Substanzen wie Cyclosporin, Cisplatin und/oder Ifosfamid.

19. Arzneimittel nach Anspruch 11 zur Prophylaxe des akuten Nierenversagens, postoperativ oder nach hypertonen Krisen sowie nach Gabe von Kontrastmitteln bei Patienten mit eingeschränkter Nierenfunktion.

20. Arzneimittel nach Anspruch 11 als Diagnostikum zur Differentialdiagnose von Endothel-Veränderungen.

21. Arzneimittel nach Anspruch 11, dadurch gekennzeichnet, daß es 10 ng bis 50 µg Urodilatin (ANF/CDD 95-126) pro Dosiseinheit enthält.

22. Arzneimittel nach Anspruch 11 mit vasodilatorischer Wirkung, dadurch gekennzeichnet, daß es Urodilatin (ANF/CDD 95-126) als Wirkstoff enthält.

23. Verfahren zur spezifischen Bestimmung von Cardiodilatin, dadurch gekennzeichnet, daß man nach dem Prinzip des Immunoassays arbeitet und einen Antikörper verwendet, der gegen Urodilatin (ANF/CDD 95-126) gerichtet ist.

24. Verwendung des Urodilatins (ANF/CDD 95-126) nach Anspruch 1 zur Herstellung eines Diagnostikums zur Differentialdiagnose von Endothel-Veränderungen.

25. Verwendung des Urodilatins (ANF/CDD 95-126) nach Anspruch 1 für die Herstellung von Arzneimitteln wie Injektions- oder Infusionslösungen zur Behandlung von akuter Niereninsuffizienz, nephrotischem und nephritischem Syndrom, terminaler Niereninsuffizienz, akuter Herzinsuffizienz, Ascites, generalisierten Ödemen, Lungenödem, Hirnödem, primären und sekundären Lymphödemen, Hydrothorax, Glaukom, Vena-cava-Stenose, Hypoproteinämie, Hörsturz, essentieller und renaler Hypertonie, maligner Hypertonie, EPH-Gestose wie Schwangerschaftshypertonie, Schwangerschafts-Nephrose, zerebralem Natriumspeichersyndrom, Gefäßspasmen wie Morbus Raynaud, Angina abdominalis, Koronarspasmen, koronarer Herzkrankheit, vasomotorischen Kopfschmerzen, Kopfschmerzen bei Hypertonie, Bing-Horton-Syndrom Migräne, Durchblutungsstörungen im Vertebralis-Basilaris-Strombahngebiet, Störungen des Renin-Angiotensin-Systems, primärem und sekundärem Hyperaldosteronismus, reninsezernierenden Tumoren, Phäochromozytom, Bartters Syndrom, Schwartz-Bartter-Syndrom, Pankreasinsuffizienz, primärer Schweißdrüseninsuffizienz wie Anhidrosis, Miktions- und Defäkationsstörungen, Hypophysenvorderlappendysregulation, cardio-vaskulären Nebenwirkungen bei der Therapie psychiatrischer Erkrankungen sowie Agitation mit Catecholaminerhöhung.

26. Verwendung des Urodilatins (ANF/CDD 95-126) nach Anspruch 1 für die Herstellung von Arzneimitteln wie Injektions- oder Infusionslösungen zur unterstützenden Behandlung bei Herztransplantationen, "PEEP-Beatmung" (einer mechanischen Beatmung mit positivem endexpiratorischem Druck) sowie Bypass-Operationen.

27. Verwendung des Urodilatins (ANF/CDD 95-126) nach Anspruch 1 für die Herstellung von Arzneimitteln wie Injektions- oder Infusionslösungen zur Behandlung von Komplikationen nach Einpflanzung eines künstlichen Herzens.

28. Verwendung des Urodilatins (ANF/CDD 95-126) nach Anspruch 1 für die Herstellung von Arzneimitteln wie Injektions- oder Infusionslösungen als Diuretikum zur Behandlung von multiplen Erkrankungen wie Gicht, kombiniert mit Hypertonus/Herzinsuffizienz sowie Diabetes mit Hypertonus/Herzinsuffizienz.

29. Verwendung des Urodilatins (ANF/CDD 95-126) nach Anspruch 1 für die Herstellung von Arzneimitteln wie Injektions- oder Infusionslösungen als Diuretikum in Kombination mit Cephalosporinen, Aminoglykosiden oder Antikoagulantien.

30. Verwendung des Urodilatins (ANF/CDD 95-126) nach Anspruch 1 für die Herstellung von Arzneimitteln wie Injektions- oder Infusionslösungen zur Prophylaxe von akutem Nierenversagen nach Nierentransplantationen und Behandlung mit nephrotoxischen Substanzen wie Cyclosporin, Cisplatin und/oder Ifosfamid.

31. Verwendung des Urodilatins (ANF/CDD 95-126) nach Anspruch 1 für die Herstellung von Arzneimitteln wie Injektions- oder Infusionslösungen zur Prophylaxe des akuten Nierenversagens, postoperativ oder nach hypertonen Krisen sowie nach Gabe von Kontrastmitteln bei Patienten mit eingeschränkter Nierenfunktion.

32. Verwendung des Urodilatins (ANF/CDD 95-126) nach Anspruch 1 für die Herstellung von Arzneimitteln wie Injektions- oder Infusionslösungen als Diagnostikum zur Differentialdiagnose von Endothel-Veränderungen.

## Revendications

1. Un peptide ayant la séquence d'acides aminés 95-126 de ANF/CDD 1-126 (gamma-hANaP) portant le nom d'urodilatine (ANF/CDD 95-126), répondant à la formule

Thr-Ala-Pro-Arg-Ser-Leu-Arg-Arg-Ser-Ser-Cys-Phe-Gly-Gly-Arg-Met-
S
S
Asp-Arg-Ile-Gly-Ala-Gln-Ser-Gly-Leu-Gly-Cys-Asn-Ser-Phe-Arg-Tyr

2. Un procédé pour préparer le fragment de cardiodilatine dénommé urodilatine (ANF/CDD 95-126) selon la revendication 1, par synthèse par étapes sur une phase solide en utilisant des acides aminés protégés par des groupes protecteurs.

3. Le procédé selon la revendication 2, caractérisé en ce que les groupes tert.-butyloxycarbonyle, fluorénylméthyloxycarbonyle et/ou 3,5-diméthoxyphényl-2,2-propyloxycarbonyle sont utilisés comme groupes protecteurs.

4. Un procédé pour préparer le fragment de cardiodilatine dénommé urodilatine (ANF/CDD 95-126) selon la revendication 1, caractérisé en ce que ANF/CDD 99-126 qui a été fixé à une phase solide est amené à réagir avec le tétrapeptide Thr-Ala-Pro-Arg, le peptide résultant est détaché du support, soumis à une cyclisation après élimination des groupes protecteurs et traité et purifié d'une manière connue en soi.

5. Un procédé pour préparer le fragment de cardiodilatine dénommé urodilatine (ANF/CDD 95-126) selon la revendication 1, caractérisé en ce que le peptide ANF/CDD 99-126 est dissous dans un solvant, le tétrapeptide Thr-Ala-Pro-Arg, muni de groupes protecteurs si nécessaire, est ajouté en même temps qu'un agent activant, le produit résultant est soumis à une chromatographie après élimination des groupes protecteurs et encore purifié d'une manière connue en soi.

6. Le procédé selon l'une quelconque des revendication 4 et 5, caractérisé en ce que le tétrapeptide utilisé est Boc-Thr(But)-Ala-Pro-Arg(Tos).

7. Un procédé pour extraire l'urodilatine (ANF/CDD 95-126) selon la revendication 1 de fluides corporels, caractérisé en ce que l'urine est utilisée comme matière de départ, de l'acide alginique est ajouté à la solution, les peptides adsorbés sur l'acide alginique sont élués, l'éluat est fractionné selon des méthodes classiques de purification biochimique, et la fraction active est identifiée par des méthodes classiques pour la détection de l'activité biologique.

8. Le procédé selon la revendication 7, caractérisé en ce que l'urodilatine (ANF/CDD 95-126) adsorbée sur l'acide alginique est éluée, l'éluat est soumis à un relargage ou est lyophilisé, le précipité est dessalé par chromatographie sur gel, l'extrait brut obtenu est soumis à une chromatographie par échange d'ions et ensuite séparé par CLHP.

9. Le procédé selon la revendication 8, caractérisé en ce que la séparation par CLHP est effectuée en utilisant un gel de silice à phase inversée.

10. Un procédé pour extraire l'urodilatine (ANF/CDD 95-126), caractérisé en ce que de l'urine est mise en contact avec de l'acide alginique, l'urodilatine (ANF/CDD 95-126) adsorbée sur l'acide alginique est éluée, l'éluat est fractionné selon des méthodes classiques de purification biochimique en utilisant un test dans lequel la fraction contenant l'urodilatine (ANF/CDD 95-126) est identifiée au moyen de son effet relaxant sur la musculature lisse.

11. Médicament, contenant de l'urodilatine (ANF/CDD 95-126) selon la revendication 1 comme ingrédient actif en association avec un support et/ou diluant pharmacologiquement compatible.

12. Le médicament selon la revendication 11, pour le diagnostic et la thérapie d'hypertension, maladies cardiaques et vasculaires, maladies de la peau y compris les troubles de la perspiration, collapsus cardiovasculaire, troubles rénaux et corticosurrénaux, maladies du tube digestif et plus particulièrement les troubles de la motilité, oedèmes cérébraux, glaucome, coronaropathies spasmodiques et maladies neurologiques.

13. Le médicament selon la revendication 11, pour le traitement d'insuffisance rénale aiguë, syndrome néphrotique et néphrétique, insuffisance rénale terminale, insuffisance coronarienne aiguë, ascites, oedèmes généralisés, oedème pulmonaire, oedème cérébral, oedèmes lymphatiques primaire et secondaire, hydrothorax, glaucome, sténose de la veine cave, hypoprotéinémie, hypoacousie, hypertension essentielle et rénale, hypertension maligne, gestose EPH telle que hypertension de grossesse, néphrose de grossesse, maladie de surcharge sodique cérébrale, spasmes vasculaires tels que maladie de Raynaud, angor abdominal, spasmes coronariens, cardiopathie ischémique, céphalée vasomotrice, céphalée par hypertension, migraine du syndrome de Bing-Horton, troubles de la circulation sanguine dans la région de la voie d'écoulement vertébro-basilaire, troubles du système rénine-angiotensine, hyperaldostéronisme primaire et secondaire, tumeurs à rénine, phéochromocytome, syndrome de Bartter, syndrome de Schwartz-Bartter, insuffisance pancréatique, insuffisance primaire des glandes sudoripares telle que l'anhidrose, troubles de la miction et de la défécation, dérèglement du lobe antérieur de l'hypophyse, effets secondaires cardio-vasculaires dans la thérapie de maladies psych(iatr)iques et agitation avec augmentation du catéchol.

14. Le médicament selon la revendication 11, pour le traitement de soutien lors de transplantations cardiaques, de "respiration PEEP" (une respiration artificielle avec pression positive en fin d'expiration) ainsi que d'opérations de pontage.

15. Le médicament selon la revendication 11, pour le traitement de complications après implantation d'un coeur artificiel.

16. Le médicament selon la revendication 11, comme diurétique pour le traitement de maladies multiples telles que la goutte combinée à l'hypertension/insuffisance cardiaque, ainsi que le diabète avec hypertension/insuffisance cardiaque.

17. Le médicament selon la revendication 11, comme diurétique en association avec des céphalosporines, aminosides ou anticoagulants.

18. Le médicament selon la revendication 11, pour la prophylaxie d'une insuffisance rénale aiguë après des greffes de rein et un traitement par des substances néphrotoxiques telles que la cyclosporine, le cisplatine et/ou l'ifosfamide.

19. Le médicament selon la revendication 11, pour la prophylaxie d'une insuffisance rénale aiguë qui peut survenir en phase postopératoire ou après une crise hypertensive, ainsi qu'après administration d'un milieu de contraste pour un patient souffrant d'une fonction limitée du rein.

20. Le médicament selon la revendication 11, comme agent diagnostique pour diagnostics différentiels de modifications endothéliales.

21. Le médicament selon la revendication 11, caractérisé en ce qu'il contient 10 ng à 50 µg d'urodilatine (ANF/CDD 95-126) par unité posologique.

22. Le médicament selon la revendication 11 ayant un effet vasodilatateur, caractérisé en ce qu'il contient de l'urodilatine (ANF/CDD 95-126) comme ingrédient actif.

23. Une méthode pour la détermination spécifique de la cardiodilatine, caractérisée en ce qu'elle opère selon le principe d'un dosage immunologique et fait usage d'un anticorps dirigé contre l'urodilatine (ANF/CDD 95-126).

24. Utilisation de l'urodilatine (ANF/CDD 95-126) selon la revendication 1, pour la préparation d'un agent diagnostique pour diagnostics différentiels de modifications endothéliales.

25. Utilisation de l'urodilatine (ANF/CDD 95-126) selon la revendication 1, pour la préparation de médicaments tels que des solutions pour injection ou perfusion pour le traitement d'insuffisance rénale aiguë, syndrome néphrotique et néphrétique, insuffisance rénale terminale, insuffisance coronarienne aiguë, ascites, oedèmes généralisés, oedème pulmonaire, oedème cérébral, oedèmes lymphatiques primaire et secondaire, hydrothorax, glaucome, sténose de la veine cave, hypoprotéinémie, hypoacousie, hypertension essentielle et rénale, hypertension maligne, gestose EPH telle que hypertension de la grossesse, néphrose de la grossesse, maladie de surcharge sodique cérébrale, spasmes vasculaires tels que maladie de Raynaud, angor abdominal, spasmes coronariens, cardiopathie ischémique, céphalée vasomotrice, céphalée par hypertension, migraine du syndrome de Bing-Horton, troubles de la circulation sanguine dans la région de la voie d'écoulement vertébro-basilaire, troubles du système rénine-angiotensine, hyperaldostéronisme primaire et secondaire, tumeurs à rénine, phéochromocytome, syndrome de Bartter, syndrome de Schwartz-Bartter, insuffisance pancréatique, insuffisance primaire des glandes sudoripares telle que l'anhidrose, troubles de la miction et de la défécation, dérèglement du lobe antérieur de l'hypophyse, effets secondaires cardio-vasculaires dans la thérapie de maladies psych(iatr)iques et agitation avec augmentation du catéchol.

26. Utilisation de l'urodilatine (ANF/CDD 95-126) selon la revendication 1 pour la préparation de médicaments tels que des solutions pour injection et perfusion pour le traitement de soutien lors de transplantations cardiaques, de "respiration PEEP" (une respiration artificielle avec pression positive en fin d'expiration) ainsi que d'opérations de pontage.

27. Utilisation de l'urodilatine (ANF/CDD 95-126) selon la revendication 1 pour la préparation de médicaments tels que des solutions pour injection et perfusion pour le traitement de complications après implantation d'un coeur artificiel.

28. Utilisation de l'urodilatine (ANF/CDD 95-126) selon la revendication 1 pour la préparation de médicaments tels que des solutions pour injection et perfusion pour le traitement de maladies multiples telles que la goutte combinée à l'hypertension/insuffisance cardiaque, ainsi que le diabète avec hypertension/insuffisance cardiaque.

29. Utilisation de l'urodilatine (ANF/CDD 95-126) selon la revendication 1 pour la préparation de médicaments tels que des solutions pour injection et perfusion comme diurétique en association avec des céphalosporines, aminosides ou anticoagulants.

30. Utilisation de l'urodilatine (ANF/CDD 95-126) selon la revendication 1 pour la préparation de médicaments tels que des solutions pour injection et perfusion pour la prophylaxie d'une insuffisance rénale aiguë après les greffes de rein et un traitement par des substances néphrotoxiques telles que la cyclosporine, le cisplatine et/ou l'ifosfamide.

31. Utilisation de l'urodilatine (ANF/CDD 95-126) selon la revendication 1 pour la préparation de médicaments tels que des solutions pour injection et perfusion pour la prophylaxie d'une insuffisance rénale aiguë qui peut survenir en phase postopératoire ou après une crise hypertensive, ainsi qu'après administration d'un milieu de contraste pour un patient souffrant d'une fonction limitée du rein.

32. Utilisation de l'urodilatine (ANF/CDD 95-126) selon la revendication 1 pour la préparation de médicaments tels que des solutions pour injection et perfusion comme agent diagnostique pour diagnostics différentiels de modifications endothéliales.
